# EUROPEAN PATENT APPLICATION

(11) **EP 4 201 397 A1**
(43) Date of publication of application: **28.06.2023**
(21) Application number: 21217436.1
(22) Date of filing: 23.12.2021
(51) Int. Cl.: A61K 9/00, A61K 9/107, A61K 47/36, A61P 31/22

(54) **YEAST BETA-GLUCAN EMULSION, METHODS AND USES THEREOF**

(30) Priority: 21.12.2021 PT 2021117662
(71) Applicant: Universidade Católica Portuguesa - UCP, 4169-005 Porto (PT); Amyris Bio Products Portugal, Unipessoal, LDA, 4169-005 Porto (PT)
(72) Inventor: CRUZ FERNANDES, JOÃO, 4300-218 PORTO (PT); FARIA AMORIM, MARIA MANUELA, 4465-026 SÃO MAMEDE DE INFESTA (PT); CONSTANTE DE SOUSA, PEDRO MIGUEL, 4470-320 MAIA (PT); TAVARES VALENTE, DIANA MARIA, 4520-038 SANTA MARIA DA FEIRA (PT); AZEVEDO SILVA, JOÃO PEDRO, 4785-321 TROFA (PT); ESTEVEZ PINTADO, MARIA MANUELA, 4169-005 PORTO (PT)
(74) Representative: Patentree

(57) **Abstract**

The present disclosure relates to a yeast β-Glucan emulsion for use in prevention or treatment of herpes simplex virus infection, in particular wherein the emulsion comprises an emollient agent, wherein the emollient agent is selected from: squalane, butter, hemp oil, or mixtures thereof.

The present disclosure relates to yeast β-Glucan emulsion with anti-herpetic activity, in particular a face topical composition, more in particular lipstick/face stick/ lip or face balm/roll on with anti-herpetic properties; method for obtaining and their use thereof.

The present disclosure also relates to a face topical composition, preferably antiherpetically active lipstick, or face stick, or an antiherpetic lip or face balm, or an antiherpetic roll on comprising β-glucans and the use for the prevention and treatment of disorders of the lips and other areas of the face caused by human herpes viruses.

## Description

### TECHNICAL FIELD

The present disclosure relates to yeast β-Glucan emulsion with anti-herpetic activity, in particular a face topical composition; more in particular a lipstick/face stick/lip or face balm/roll on with anti-herpetic properties; method for obtaining and their use thereof.

The present disclosure also relates to a face topical composition, preferably antiherpetically active lipstick, or face stick, or an antiherpetic lip or face balm, or an antiherpetic roll on comprising β-glucans and the use for the prevention and treatment of disorders of the lips and other areas of the face caused by human herpes simplex viruses.

### BACKGROUND

Glucans are polysaccharides present in different organisms, from plants to fungi such as mushrooms or even yeast. In Saccharomyces species, glucans are a major constituent of the cell wall. β-glucans, are a specific type of glucans composed of d-glucose units linked by glycosidic bonds β-(1-3); (1-6). This specific type of glucans has interesting biological activities. Many studies have documented the β-glucans activity such as anti-wrinkle activity, antioxidant activity and hydration, skincare application; tissue regeneration, activity against viral infections, and immunostimulatory activities such as pro-inflammatory effects. β-glucans are able to trigger immune responses by interacting with pattern recognition receptors expressed by immune cells. One of these cases is the capacity of β-glucans to trigger innate immunity response by interacting with dectin-1 receptor.

These facts are disclosed in order to illustrate the technical problem addressed by the present disclosure.

### GENERAL DESCRIPTION

The present disclosure relates to antiherpetic emulsion comprising β-glucans and the use thereof for the prevention and treatment of disorders of the lips and other areas of the face caused by human herpes simplex viruses.

An aspect of the present disclosure relates to a yeast β-Glucan emulsion for use in prevention or treatment of herpes simplex virus infection, wherein the emulsion comprises an emollient agent, preferably wherein the emollient agent is selected from: squalane, butter, hemp oil, or mixtures thereof.

In an embodiment, the yeast β-Glucan emulsion of the present disclosure may be used in the prevention and treatment of labial diseases or other areas of the face of the lip affected by human herpes viruses.

In an embodiment, the yeast β-Glucan emulsion of the present disclosure may be administrated in the form of a face topical composition; preferably in the form of an antiherpetic lipstick, face stick, lip balm, face balm, or roll on.

In an embodiment, the yeast β-Glucan emulsion of the present disclosure is insoluble in water.

In an embodiment, the yeast β-Glucan emulsion of the present disclosure may be a native β-Glucan.

In an embodiment, the yeast cell may be *Saccharomyces cerevisiae; Pichia pastoris, Cyberlindnera jadinii, Candida albicans,* or mixtures thereof. In a preferred embodiment, the yeast glucans may be extracted from spent yeast from S. cerevisiae. Preferably *Saccharomyces* strain CEN.PK2 Genotype: MATa/α ura3-52/ura3-52 trp1-289/trp1-289 leu2-3,112/leu2-3,112 his3 Δ1/his3 Δ1 MAL2-8C/MAL2-8C SUC2/SUC2, CEN.PK possesses a mutation in CYR1 (A5627T) corresponding to a K1876M substitution near the end of the catalytic domain in adenylate cyclase which eliminates glucose- and acidification-induced cAMP signaling and delays glucose-induced loss of stress resistance).

In an embodiment, the amount of the β-Glucan may range from 1-40% (w/w); preferably 5-25% (w/w).

In an embodiment, the molecular weight of β-Glucan ranges from 200 - 800 KDa, preferably 300 - 600 KDa.

In an embodiment, the emollient agent amount may range from 30-80 % (w/w); preferably 60 - 80 % (w/w).

In an embodiment, the emollient agent is squalane.

Another aspect of the present disclosure relates to a facial topical composition comprising a yeast β-Glucan emulsion wherein the emulsion comprises an emollient agent; preferably wherein the emollient agent is selected from: squalane, butter, hemp oil, or mixtures thereof; for use in the prevention or treatment of herpes simplex virus infection, wherein said composition is administered topically in the face; in particular in the form of an antiherpetic lipstick, face stick, lip balm, face balm, or a roll on.

In an embodiment, the facial topical composition of the present disclosure may further comprise further comprising at least one of the following compounds: at least a filler; at least an oil (preferably an essential oil), at least a preservative, at least a dye, at least an aroma, at least a flavour, at least an hydrating agent; at least a pH adjuster; at least a stabilizer; a thickener, or combinations thereof.

In an embodiment, the filler agent amount ranges from 10-30% (w/w), preferably 10- 20 % (w/w)

In an embodiment, the filler is selected from: waxes, paraffin, beewax, or mixtures thereof.

In an embodiment, the preservative is selected from: citric acid, phenethyl alcohol, benzoic acid, sorbic acid, salicylic acid alcohol, tocopherol, or mixtures thereof.

In an embodiment, the preservative amount ranges from 0.1-2 %(w/w), preferably 0.5- 1 % (w/w)

In an embodiment, the weight ratio glucan/emollient agent ranges from 0.5:1 - 1:2; preferably 1:1 - 1:2.

In an embodiment, the facial topical composition of the present disclosure may comprise: a dye, an aroma, a flavour, a hydrating agent; a pH adjuster; a stabilizer; a thickener, or combinations.

Another aspect of the present disclosure relates to a method of obtaining β-Glucan of the present disclosure comprising the following steps:
obtaining autolyzed yeasts cells;
extract dried alkali-glucans of the yeast by adding an alkaline solution to the yeast cells in a solid-liquid extraction and collecting a first pellet;
adding to the obtained first pellet an alcoholic solution and an acid solution in a second solid-liquid extraction to obtain a second pellet.

### BRIEF DESCRIPTION OF THE DRAWINGS

The following figures provide preferred embodiments for illustrating the disclosure and should not be seen as limiting the scope of invention.
**Figure 1****:** Schematic representation of an embodiment of HaCat cells infected with HSVI and exposed for 48h to glucans. Cell death is expressed in comparison to cells infected with HSVI. Acyclovir was used as a positive control.
**Figure 2****:** Schematic representation of an embodiment of the production of IL-1β, IL-8 and TNF-α cytokines in macrophages derived from THP-1 cells infected with HSVI.
**Figure 3****:** Schematic representation of an embodiment of a visual appearance glucan powder (A) and glucan solution (aqueous) and emulsified with squalane (B).
**Figure 4****:** Schematic representation of an embodiment of a Crystal Violet staining of HaCaT cells infected with HSVI and treated with β-glucans and β-glucans formulated with squalane.
**Figure 5****:** Representative graphs of frequency vs intensity of β-glucans fluorescence in skin section tissues stained with calcofluor for treatment with Glu-RbM or Glu-RbM/SQ for 2h (A) and Glu-RbM/SQ 2h and 6h (B).

### DETAILED DESCRIPTION

The present disclosure relates to a yeast β-Glucan emulsion for use in prevention or treatment of herpes simplex virus infection, in particular wherein the emulsion comprises an emollient agent, wherein the emollient agent is selected from: squalane, butter, hemp oil, or mixtures thereof.

The present disclosure relates to yeast β-Glucan emulsion with anti-herpetic activity, in particular a face topical composition, more in particular lipstick/face stick/ lip or face balm/roll on with anti-herpetic properties; method for obtaining and their use thereof.

The present disclosure also relates to a face topical composition, preferably antiherpetically active lipstick, or face stick, or an antiherpetic lip or face balm, or an antiherpetic roll on comprising β-glucans and the use for the prevention and treatment of disorders of the lips and other areas of the face caused by human herpes viruses.

The present disclosure relates to a formulation of a lipstick/ lip balm/ roll on containing β-Glucans with anti-herpetic activity, thus a lipstick/lip balm/ roll on with anti-herpetic properties.

List of abbreviations:
**Glu-RbM** - Glucan Alkali+Organic/acid treatment from spent RebM producing yeast
**Glu-WT** - Glucan Alkali+Organic/acid treatment from wild type yeast CEN.PK2

Wild type yeast CEN.PK2 - Saccharomyces *cerevisiae* strain CEN.PK2. CEN.PK2 Genotype: MATa/α ura3-52/ura3-52 trp1-289/trp1-289 leu2-3,112/leu2-3,112 his3 Δ1/his3 Δ1 MAL2-8C/MAL2-8C SUC2/SUC2, CEN.PK possesses a mutation in CYR1 (A5627T) corresponding to a K1876M substitution near the end of the catalytic domain in adenylate cyclase which eliminates glucose- and acidification-induced cAMP signaling and delays glucose-induced loss of stress resistance;
**RbM** -RebM sweetener molecule;
**HaCat** -human epidermal keratinocyte cells;

### Glucans Extraction and Purification

In an embodiment, we have extracted glucans from spent yeast engineered to produce the sweetener molecule - RebM (RbM). As these strains are genetic modified organism (GMO), we are also testing glucans from the wild-type Saccharomyces *cerevisiae* strain CEN.PK2. In an embodiment, yeast glucans were extracted from spent yeast from the production of sweetener molecule. These strains may be genetically modified organism (GMO), glucans from the wild-type *Saccharomyces cerevisiae* strain CEN.PK2 was also extracted. *Saccharomyces cerevisiae* strain CEN.PK2 Genotype: MATa/α ura3-52/ura3-52 trp1-289/trp1-289 leu2-3,112/leu2-3,112 his3 Δ1/his3 Δ1 MAL2-8C/MAL2-8C SUC2/SUC2, CEN.PK possesses a mutation in CYR1 (A5627T) corresponding to a K1876M substitution near the end of the catalytic domain in adenylate cyclase which eliminates glucose- and acidification-induced cAMP signaling and delays glucose-induced loss of stress resistance.

In an embodiment, the yeasts were subjected to a heat treatment in order to release cellular components - autolysis, enabling the isolation and purification of the glucans present in the insoluble fraction (pellet) of the yeast cell wall polysaccharides. After autolysis, the first step in the extraction was initiated by an alkaline treatment, where an initial 20% (w/v) solution was prepared using autolyzed yeast pellet and sodium hydroxide (NaOH 1M) as a solvent. Then, this solution was placed in a water bath at 90 °C for 2-4 hours. After this, it was centrifuged at 8000 rpm, for 10 min at 4 °C and the supernatant was discarded. The resulting pellet washed three times by centrifugation with deionized water. The washed pellet was re-suspended in 50 mL of deionized water and neutralized until pH 7 with hydrochloric acid (HCI 3M). The supernatant was removed by centrifugation and a last wash was done using the same volume of deionized water. The resulting pellet, containing insoluble alkali-glucans, was completely homogenized in deionized water and dried by spray dry, at an inlet temperature of 110 °C and an outlet of 50 °C.

In order to remove remaining proteins, lipids and other unwanted compounds, an acid/ethanol extraction was used. For this extraction, 1 g of dried alkali-glucans was dissolved in 80 ml of ethanol (99%) and 1,6 ml of HCI 32% (w/v) and placed at 50 °C during 4 hours in an orbital shaker. Then, the pellet was washed three times - twice with absolute ethanol and one with acetone - suspending 20 ml of each solvent and centrifuge at 4 °C, 8000 rpm for 10 min. Purified glucans was dried in a vacuum oven at 50 °C overnight.

### β-Glucans protect against HSVI infection

The beneficial effects of β-glucans as inflammatory modulators are extensively studied. They have the capacity to trigger immunologic responses by interacting with specific macrophages receptors. Some studies have pointed that β-glucans present antiviral capacity.

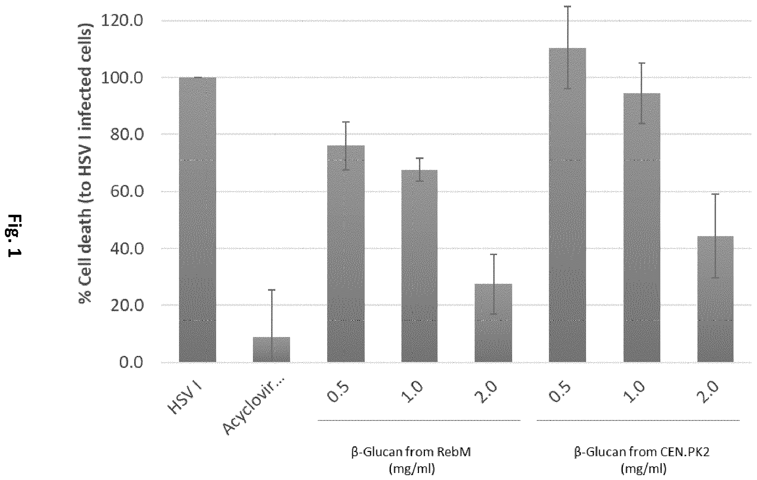

We have tested the antiviral capacity of Glu-RbM and Glu-WT extracts, in human epidermal keratinocyte (HaCat) infected with HSVI. HaCaT cells were seeded in 96-well plates at 1×10⁵ cells/ml. After, cells were exposed to HSVI at a multiplicity of infection (MOI) of 0.2 in 199V medium for 1h at 37 °C. Media with virus was removed and fresh medium was added alone or supplemented with different glucans concentrations. After 48h of exposure, Presto blue assay was performed and the results were expressed as cell death percentage in comparison to cells infected with HSVI. Our results indicates that Glu-Rb and Glu-WT protect keratinocytes from HSVI infection in a dose dependent manner (Figure 1). These results were replicated in other cell lines such as Vero cells and macrophages derived from THP-1 cells (data not shown).

### β-Glucans stimulate the immune system in HSVI infection

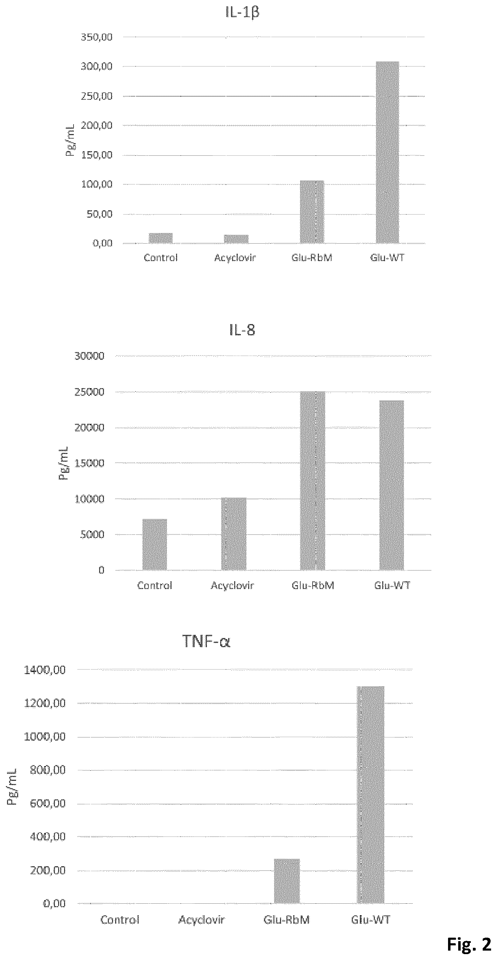

In an embodiment, in order to study the immunomodulatory effect of β-glucans in cells infected with HSVI, THP-1 cells were seeded in 24-well plates at 3.5×10⁵ cells/ml and exposed to phorbol-12-myristate-13-acetate (PMA, 50nM) for 72h to induce macrophage differentiation. Then, cells were infected with HSVI and treated with different glucans for 48h. The immunomodulatory activity was evaluated by flow cytometry using a BioLegend's LEGENDplex^{™} bead-based immunoassay. Our results indicate that β-glucans induce the expression of IL-1β, IL-8 and TNF-α in HSVI cells indicating that β-glucans stimulate the immune system to counteract HSVI infection (Figure 2).

### β-Glucans formulated with emollients retain anti-herpetic activity

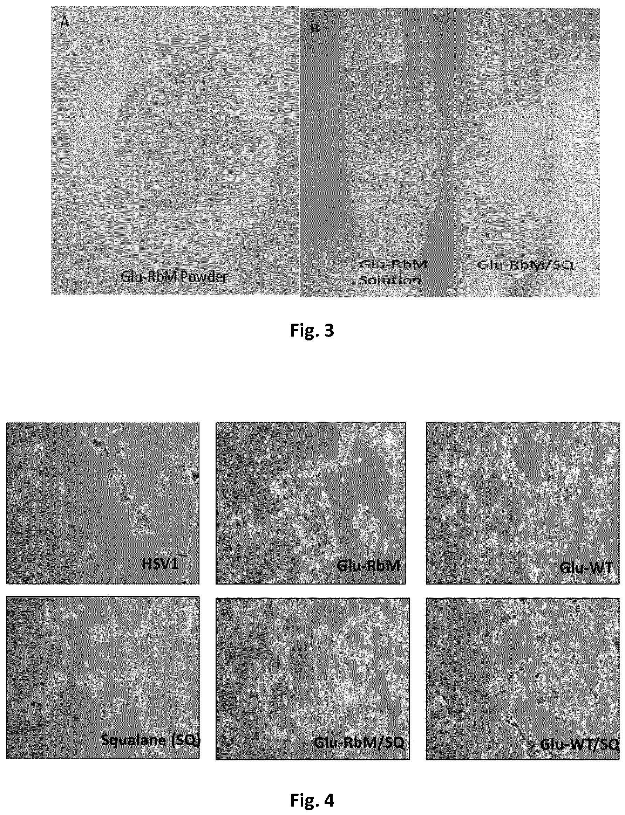

In an embodiment, β-glucans are insoluble, thus to be used in a formulation they must be solubilized. To achieve this, we have mixed glucans with emollient agents such as squalane in a proportion of 1:1 (w/w) (Figure 3). Other agents such as squalane, butter, hemp oil, or mixtures thereof, preferably squalane.

To verify if the formulated β-glucans retain its anti-herpetic capacity, HaCaT cells were seeded in 24-well plates at 3×10⁵ cells/well. The cells were then infected with HSVI and treated with β-glucans for 48h. Then the cells were washed and fixed with 4% Paraformaldehyde (PFA) for 20 minutes and stained with crystal violet at 0.05%. After extensive washing and air-dried, microscope images of the different conditions were taken in an inverted microscope (Figure 4). The results indicate that formulated glucans are able to prevent HSVI induced cell death, similar to β-glucans alone.

### Emollient increases permeability of glucans in the skin

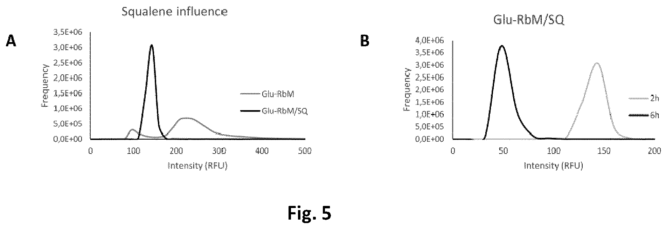

In an embodiment, the formulation of β-glucans with emollients such as squalane lead also to increased permeability properties. Due to the insolubility of β-glucans, when in contact with the skin they will not penetrate easily, thus not interacting with skin cells. This leads to a decrease in β-glucans activity. However, when formulated with squalane, β-glucans are able to better penetrate in the skin. In order to test β-glucans permeability skin grafts were exposed to β-glucans and β-glucans formulated with squalane were used to treat skin grafts using a Franz diffusion cell methodology. Skin grafts were processed for histological analysis. Briefly, skin grafts were submitted to 4 main steps: fixation with PFA 4%, dehydration, clearing and finally embedding in paraffin. Tissue sections (4µm) were stained with Hematoxilin & eosin for tissue structure evaluation and stained with a specific fluorescent dye for β-glucans, calcofluor (with KOH at 1:1). The microscope fluorescence images of samples stained with calcofluor were evaluated by intensity/frequency analysis. The results show that at 2h of exposure, Glu-RbM/SQ as a wider distribution (improved frequency) in the skin than Glu-RbM alone (Fig. 5A). After 6h, squalane improved the frequency (distribution) of glucans in skin tissue in comparison to the 2h (Figure 5B).

### β-glucans incorporation in a lipstick balm

In an embodiment, in order to incorporate β-glucans in a formulation to be used as a lipstick, a lip balm, or a roll-on the following composition in weight percent was developed:
Structural agents (or filler): 10 - 20% (w/w);
Emollients: 60 - 80% (w/w);
Glucan: 1 - 40% (w/w);
Preservative: 0.1- 1% (w/w).

In an embodiment, waxes were used as structuring agent or as filler , particularly soy, rice and candelilla waxes. The emollients acts as moisturizing agents to soothe and hydrate the skin and were included on the form of butter, such shea and cocoa butter, and oils - coconut, sweet almond oils and squalane. The yeast β-Glucan of the present disclosure (5-25 % w/w) were added in emulsion with squalane to improve skin penetration. To ensure stability and prevent lipid degradation, tocopherol was added as preservative. This formulation can be considered vegan.

The term "comprising" whenever used in this document is intended to indicate the presence of stated features, integers, steps, components, but not to preclude the presence or addition of one or more other features, integers, steps, components or groups thereof.

Where singular forms of elements or features are used in the specification of the claims, the plural form is also included, and vice versa, if not specifically excluded. For example, the term "a β-Glucan" or "the β-Glucan" also includes the plural forms "P-Glucans" or "the β-Glucans," and vice versa. In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention also includes embodiments in which more than one, or all of the group members are present in, employed in, or otherwise relevant to a given product or process.

Furthermore, it is to be understood that the invention encompasses all variations, combinations, and permutations in which one or more limitations, elements, clauses, descriptive terms, etc., from one or more of the claims or from relevant portions of the description is introduced into another claim. For example, any claim that is dependent on another claim can be modified to include one or more limitations found in any other claim that is dependent on the same base claim.

Furthermore, where the claims recite a composition, it is to be understood that methods of using the composition for any of the purposes disclosed herein are included, and methods of making the composition according to any of the methods of making disclosed herein or other methods known in the art are included, unless otherwise indicated or unless it would be evident to one of ordinary skill in the art that a contradiction or inconsistency would arise.

Where ranges are given, endpoints are included. Furthermore, it is to be understood that unless otherwise indicated or otherwise evident from the context and/or the understanding of one of ordinary skill in the art, values that are expressed as ranges can assume any specific value within the stated ranges in different embodiments of the invention, to the tenth of the unit of the lower limit of the range, unless the context clearly dictates otherwise. It is also to be understood that unless otherwise indicated or otherwise evident from the context and/or the understanding of one of ordinary skill in the art, values expressed as ranges can assume any subrange within the given range, wherein the endpoints of the subrange are expressed to the same degree of accuracy as the tenth of the unit of the lower limit of the range.

The disclosure should not be seen in any way restricted to the embodiments described and a person with ordinary skill in the art will foresee many possibilities to modifications thereof.

The above described embodiments are combinable.

The following claims further set out particular embodiments of the disclosure.

## Claims

1. A yeast β-Glucan emulsion for use in prevention or treatment of herpes simplex virus infection, wherein the emulsion comprises an emollient agent.

2. The yeast β-Glucan emulsion according to the previous claim wherein the emollient agent is selected from: squalane, butter, hemp oil, or mixtures thereof; preferably squalene.

3. The yeast β-Glucan emulsion according to the previous claim for use in the prevention and treatment of labial diseases or other areas of the face of the lip affected by human herpes viruses.

4. The yeast β-Glucan emulsion for use according to the previous claims wherein said emulsion is administrate in the form of a face topical composition; preferably in the from of an antiherpetic lipstick, face stick, lip or face balm, or roll on.

5. The yeast β-Glucan emulsion for use according to the previous claims wherein said yeast β-Glucan is insoluble in water, preferably wherein said yeast β-Glucan is a native β-Glucan.

6. The yeast β-Glucan emulsion for use according to any of the previous claims wherein the β-glucan amount ranges from 1-40% (w/w); preferably 5-25% (w/w).

7. The yeast β-Glucan emulsion for use according to any of the previous claims wherein the molecular weight of β-Glucan ranges from 200 - 800 KDa, preferably 300 - 600 KDa.

8. The yeast β-Glucan emulsion for use according to any of the previous claims wherein the emollient agent amount ranges from 30-80 % (w/w); preferably 60 - 80 % (w/w).

9. A facial topical composition comprising a yeast β-Glucan emulsion wherein the emulsion comprises an emollient agent, for use in the prevention or treatment of herpes simplex virus infection, wherein said composition is administrated topically in the face; in particular in the form of an antiherpetic lipstick, or face stick, or a lip or face balm, or a roll on.

10. The topical composition according to the previous claim wherein the emollient agent is selected from: squalane, butter, hemp oil, or mixtures thereof.

11. The topical composition according to any of the previous claims further comprising at least one of the following compounds: at least a filler; at least an oil (preferably an essential oil), at least a preservative, at least a dye, at least an aroma, at least a flavour, at least an hydrating agent; at least a pH adjuster; at least a stabilizer; a thickener, or combinations thereof.

12. The topical composition according to any of the previous claims wherein the filler is selected from: waxes, paraffin, beewax, or mixtures thereof; and /or wherein the preservative is selected from: citric acid, phenethyl alcohol, benzoic acid, sorbic acid, salicylic acid alcohol, tocopherol, or mixtures thereof.

13. The topical composition according to the previous claim wherein:
the filler agent amount ranges from 10-30% (w/v), preferably 10 - 20 % (w/v);
and/or; the preservative amount ranges from 0.1-2 %(w/w), preferably 0.5- 1 % (w/w).

14. The topical lipstick composition according to any of the previous claims wherein the weight ratio β-glucan/emollient agent ranges from 0.5:1 - 1:2; preferably 1:1 - 1:2.

15. Method of obtaining β-Glucan comprising the following steps:
obtaining autolyzed yeasts cells;
extract dried alkali-glucans of the yeast by adding an alkaline solution to the yeast cells in a solid-liquid extraction and collecting a first pellet;
adding to the obtained first pellet an alcoholic solution and an acid solution in a second solid-liquid extraction to obtain a second pellet;
